# EUROPEAN PATENT SPECIFICATION

(11) **EP 1 880 009 B1**
(45) Date of publication and mention of the grant of the patent: **15.06.2011**
(21) Application number: 06733568.7
(22) Date of filing: 14.04.2006
(51) Int. Cl.: C12N 15/81, C12P 21/00, C12R 1/85

(54) **HIGHLY PRODUCTIVE RECOMBINANT YEAST STRAINS WITH MODIFIED GALACTOSE-REGULATED TRANSCRIPTION**
HOCHPRODUKTIVE REKOMBINANTE HEFESTÄMME MIT MODIFIZIERTER GALACTOSE-REGULIERTER TRANSKRIPTION
SOUCHES DE LEVURE DE RECOMBINAISON A FORT RENDEMENT ET A MODIFICATION DE LA TRANSCRIPTION REGULEE PAR LA GALACTOSE

(30) Priority: 05.05.2005 SI 200500131
(43) Date of publication of application: 23.01.2008
(73) Proprietor: Kemijski Institut, 1000 Ljubljana (SI)
(72) Inventor: NOVAK STAGOJ, Mateja, 1000 Ljubljana (SI); COMINO, Sasa, 1000 Ljubljana (SI); KOMEL, Radovan, 1000 Ljubljana (SI)
(74) Representative: Grünecker, Kinkeldey, Stockmair & Schwanhäusser Anwaltssozietät
(86) International application number: PCT/SI2006/000011
(87) International publication number: WO 2006/118550

(56) References cited:
- EP-B1- 0 252 737
- KIM M -D ET AL: "Production of antithrombotic hirudin in GAL1-disrupted Saccharomyces cerevisiae" APPLIED MICROBIOLOGY AND BIOTECHNOLOGY, vol. 65, no. 3, August 2004 (2004-08), pages 259-262, XP002407629 ISSN: 0175-7598
- STAGOJ ET AL: "A novel GAL recombinant yeast strain for enhanced protein production" BIOMOLECULAR ENGINEERING, ELSEVIER, NEW YORK, NY, US, vol. 23, no. 4, September 2006 (2006-09), pages 195-199, XP005588958 ISSN: 1389-0344
- STAGOJ M N ET AL: "Fluorescence based assay of GAL system in yeast Saccharomyces cerevisiae" FEMS MICROBIOLOGY LETTERS, AMSTERDAM, NL, vol. 244, no. 1, 1 March 2005 (2005-03-01), pages 105-110, XP004759135 ISSN: 0378-1097
- OSTERGAARD SIMON ET AL: "Metabolic engineering of Saccharomyces cerevisiae" MICROBIOLOGY AND MOLECULAR BIOLOGY REVIEWS, vol. 64, no. 1, March 2000 (2000-03), pages 34-50, XP002407628 ISSN: 1092-2172

## Description

### Technical Field of the Invention

The present invention is directed to highly productive recombinant yeast strains with modified galactose-regulated transcription, to a method for producing bioactive substances, and to their use. The invention relates to the field of biotechnology. The fundamental goal of biotechnology is the industrial utilization of biocultures (microorganisms, tissue and cell cultures), to obtain various biotechnical products, useful for food industry, and pharmaceutical industry. The yeast *Saccharomyces cerevisiae* is an important host for production of a variety of heterologous proteins that have been used for the production of therapeutic and diagnostic agents, and as vaccines. *Saccharomyces cerevisiae* is considered as a GRAS (Generally Regarded as Safe) organism. In addition to safe use, the yeast *Saccharomyces cerevisiae* is the most thoroughly investigated eukaryotic microorganism.

### Summary of the Invention

The object of this invention is a microbiological method, wherein the microbiological material is subjected to manipulation. The salient feature of this invention is the replacement of the *GAL1* gene with the *GAL4* gene in the chromosome of the yeast *Saccharomyces cerevisiae.* The locus of the *GAL1* gene encoding the galactokinase enzyme is, by means of homologous recombination, replaced with the gene for the Gal4 transcription activator. The designed gene replacement causes in the mutant S. *cerevisiae* strain a significantly improved synthesis of recombinant proteins, expressed under the control of the *GAL1* promoter.

The overall procedure of *GAL* genes replacement in the genome, which is the object of this invention, yields highly productive recombinant S. *cerevisiae* strains that are applicable in the expression of industrially (pharmaceutically) interesting proteins. By the utilization of the recombinant strain, which is an object of this invention, the inputs are significantly diminished (galactose is a relatively expensive inductor), and simultaneously a high level production of the desired recombinant protein is achieved.

### State of the Art

The yeast *S*. *cerevisiae* represents an important tool for the production of recombinant proteins. Recombinant yeasts were used in the production of human interferon for the first time in 1981. One year later, recombinant vaccine for hepatitis B was produced, for the first time, with the aid of yeasts. Today, Insulin obtained from recombinant *S*. *cerevisiae* supplies half of the world's needs.

Gene expression is a complex, multi-stage process. Among the main factors, affecting the specific productivity, are promoter strength, plasmid copy number, and the presence of the 5'- untranslated leader sequences. Comprehensive research on gene regulation in yeasts considerably contributed to the understanding of gene expression mechanism in higher eukaryots. Best-investigated mechanisms in yeasts comprise, with certainty, the GAL genes regulatory mechanism. The major features of the GAL model were established by Douglas and Hawthorne in the seventies.

*GAL1, GAL7,* and *GAL10* genes are clustered near the centromere of chromosome II, while *GAL4, GAL80,* and *GAL3* regulatory genes are located on separate chromosomes. The *GAL4* gene is located on chromosome XVI. The Gal4 protein plays a key role in the regulation of *GAL* genes, as it is required for the expression of *GAL1*, *GAL2, GAL7,* and *GAL10* genes. Consequently, the mutation in *GAL4* prevents the transcription of said genes. Tightly regulated *GAL1, GAL7,* and *GAL10* genes encode proteins (galactokinase, transferase, and epimerase), crucial to galactose metabolism. An important enzyme in galactose metabolism is galactokinase (EC 2.7.1.6), catalyzing the conversion of the inductor galactose into galactose-1-phosphate. Galactose is transported into the yeast cell with a specific permease, encoded by the *GAL2* gene. The expression of *GAL* genes is precisely regulated: in the presence of galactose the expression of these genes is highly enhanced, even up to 1000-fold, while glucose acts as a potent repressor (inhibitor) on the expression thereof. The growth in a glucose containing medium inhibits, even in the presence of galactose, the expression of *GAL* genes. This phenomenon is termed catabolite repression.

Promoter sequences of *GAL1, GAL7,* and *GAL10* genes regulating high expression of said genes belong to the best-investigated in *S*. *cerevisiae.* They are important for the design and construction of vectors. Known are two crucial advantages of the utilization of *GAL* promoters. In the first place, the possibility to grow cells on a nutrient medium where the activity of both promoters is practically repressed, which enables an appropriate cell density in the culture (biomass), and successive induction. Thus, the production of proteins, potentially toxic for the yeast cell, becomes feasible. Secondly, such promoters are among the strongest inducible promoter in yeast *S*. *cerevisiae.*

A positive regulator is the Gal4 transcription factor , which is bound to promoter sequences of these genes. In the presence of glucose, the negative regulator Gal80 represses the induction by means of binding to *GAL4.* The expression of Gal4 in the cell is minimized to only one to two molecules. For an optimal induction of *GAL* promoters, a sufficient Gal4 protein level in the cell is, however, indispensable. This is especially important for the production of heterologous proteins expressed from multi-copy plasmids.

The expression level of the target protein is to a large extent determined by inherent characteristics of the heterologous protein. The yield, however, may be considerably improved by taking into account various factors influencing gene expression, and product stability. In scientific literature are disclosed several attempts of genetic engineering of the *GAL* system, with the aim to optimize fermentation processes.

Since the Gal4 transcription factor is present in very low concentrations in the cell, whereby the *GAL* genes induction is limited, researchers attempted to surmount this obstacle. The solution is not a constitutive over-expression of *GAL4,* as it results in the loss of regulation. To avoid this drawback, a controlled expression is indispensable. Laughon et al. described in the journal Molecular and Cellular Biology 4, 268-275 in 1984, transformed yeast strains that contained multi-copy plasmids, with the *GAL4* structural gene fused to the *GAL1* promoter. The scientific literature and patent specifications disclose examples for the integration of *GAL4* with the *GAL10* promoter in the yeast *S*. *cerevisiae* genome. US5068185 describes the integration of the *GAL10-GAL4* expression cassette into the *HIS3* chromosomal locus. A three-fold increase in production of the target protein was observed in the presence of galactose (US5068185), (Schultz *et al*., 1987).

The use of productive strains mutated in *GAL1* gene was disclosed by Hovland et al. in 1989 in the journal Gene, 15, 57-64, and later by several others, like Kim et al. in 2004 in the journal Applied Microbiology and Biotechnology, 65, 259-269, who disclosed an increase in the production of antithrombotic hirudin in a *GAL1*-disrupted *S*. *cerevisiae.*

They found that in mutant strains (*gal1*)*,* incapable of metabolic transformation of the inductor, the target genes expression driven by galactose-regulated promoters was significantly enhanced. In *gal1* strains, the galactose level in fermentation cultures remains unaltered, enabling and warranting a steady and constant induction of *GAL* promoters.

We solved the problem of state of the art processes, which required high galactose levels in the culture medium for the production of bioactive substances, with gene replacement in a manner enabling an enhanced production of bioactive substances required lower inductor concentrations in the culture medium. In the state of the art we found no description of the replacement of the *GAL1* gene with the *GAL4* gene, which is the object of this invention. To investigate and determine, whether the described replacement influences the heterologous gene expression, we followed the levels of green-fluorescent protein (GFP), and hepatitis B antigen (HBsAg) in the mutant strain, which is another object of this invention, in the *gal1* strain, and in the isogenic strain.

GFP has been successfully expressed in several heterologous organisms (Yang et al., 1996, Nature Biotechnology 14, 1246-1251). The gene for GFP was isolated from the jellyfish *Aequorea victoria.* The protein comprises 238 amino acids, and has a molecular mass of 26.9 kDa (Prasher al., 1992, Gene 111, 229-233). The protein structure and the fluorophore protection in its interior, are responsible for the protein stability, and its resistance against various physical and chemical agents. The principal advantage of GFP, in comparison with other reporter molecules, is the presence of the fluorophore inside the primary protein structure. Fluorescence is species-nonspecific, and stable. No cofactors or exogenous substrates are required. Fluorescence is easily detectable by UV light, and monitored non-invasively in live cells (Kain et a/., 1995, Biotechniques 19, 650-655). GFP exhibits an absorbance peak at 395 nm, a lower peak at 475 nm, and an emission (fluorescence) peak at 509 nm.

GFP is widely used in numerous fields of biotechnology. The easily performed control of the complete bioprocess: starting with clone selection, the control of production conditions, and protein isolation, enables the optimization of all working steps in the production of recombinant protein (Albano et al., 1998, Biotechnology Progress 14, 351-354**;** Misteli and Spector, 1997, Nature Biotechnology 15, 961-964**;** Poppenborg et al., 1997, Journal of Biotechnology 58, 79-88**).**

For all numerous new expression systems, the yeast S. *cerevisiae* remains an important host for obtaining various heterologous proteins utilized in the preparation of pharmaceuticals, diagnostics, and vaccines.

An obvious drawback of solutions, described in the state of the art, is that known methods of producing bioactive substances by means of galactose-regulated strains require a higher level of galactose in the nutrient medium, and provide a lower yield of the recombinant product.

The goal of this invention is, accordingly, the construction of a highly productive recombinant strain, which enables a high production of the desired recombinant protein, at low galactose levels in the culture. The recombinant strain, which is the object of this invention, makes feasible a significant increase of the yield of the complete bioprocess, and consequently, lower costs.

In conformance with this invention, this goal is achieved by the replacement of the gene encoding the enzyme for the transformation of the transcription inductor - galactose, into an inactive product, with a gene encoding the transcription factor for the galactose-inducible promoter, as claimed in the independent claims.

### Description of the Figures

FIG. 1 GFP production from the YCpGAL1-GFP vector (1 ml culture, cell density 2x10⁷/ml) at described culture conditions, in the mutant strain, in *gal1,* and in the isogenic strain.
FIG. 2 GFP production from the YEp181GAL1-GFP vector (1 ml culture, cell density 2x10⁷/ml) at described culture conditions, in the mutant strain, in *gal1,* and in the isogenic strain.

### Definitions

The term "galactose-regulation" refers to the transcription regulation of certain genes by means of effector molecules, or transcription inductors, in the present case by means of galactose.

The term "chromosome" refers to filamentous chromatin structures in the nucleus of eukaryotic cells. They act as carriers of genetic information - inscribed in linear arranged genes.

The term "transcription inductor" refers to an organic molecule inducing the transcriptional activation of structura' genes in the cell.

The term "transcription factor" refers to a gene, and to a gene product respectively, regulating the extent and rate of transcription for other, distant genes. In the present case, it is Gal4 regulating the gene transcription of *GAL* regulon.

The term "promoter" refers to a specific sequence of the DNA molecule - DNA binding site for RNA polymerase II, and other proteins, and molecules required for the transcription initiation.

The term "galactose-inducible promoter" refers to the specific sequence of the DNA molecule - DNA binding site for RNA polymerase II, and other proteins, and inductor galactose required for the transcription initiation.

The term "homologous expression" refers to the expression of a particular organism's own genetic substance or gene inscription. The product is identical to this organism with respect to the structure and activity.

The term "heterologous expression" refers to the expression of a genetic substance or gene inscription, derived from different species, in a specific host organism.

The term "host plasmid" refers to a nonchromosomal genetic element comprising a functional replication site, a genetic marker enabling its recognition, and a structural gene under the control of a strong promoter, enabling the expression of the target protein in a specific host organism.

### Description of the Invention

This invention is directed to the replacement of the *GAL1* gene with the *GAL4* gene on the chromosome in the yeast *S*. *cerevisiae.* The locus of the *GAL1* gene encoding the enzyme galactokinase is, by means of homologous recombination, replaced with the gene for the Gal4 transcription activator. Surprisingly, this enhances the synthesis of recombinant proteins expressed under the control of the *GAL1* promoter.

The above replacement relates to strains endowed with galactose-regulation, in which on the chromosome the gene encoding an enzyme that inactivates inductor galactose, has been replaced with a transcription activator of genes driven by galactose-inducible promoter. These strains are preferentially of the families *Saccharomycetaceae* or *Cryptococcaceae,* preferentially of the genus *Saccharomyces,* preferentially the *Saccharomyces cerevisiae* species.

The above strains are strains, in which the gene encoding an enzyme for the transformation of the transcription inductor into an inactive product is the *GAL1* gene.

The above strains are strains, in which the gene encoding the transcription factor is the *GAL4* gene. Typically, the synthesis of the *GAL4* transcription factor is under the control of the galactose-inducible promoter, preferably the *GAL1, GAL2, GAL7, GAL10* or *MEL1,* preferably *GAL1* promoter.

The above strains are strains, characterized in that the galactose-inducible promoter drives the expression of proteins in the presence of a transcription inductor. Preferably, these are *GAL1, GAL2, GAL7, GAL10* or *MEL1* gene promoters.

The above strains are strains, characterized in that they are hosts for plasmids, used in heterologous or homologous gene expression, and/or are used in the synthesis of bioactive substances or other products of biotechnology. Typically, plasmids used for the heterologous or homologous gene expression, are preferably under the control of the galactose-inducible promoter, preferably the *GAL1, GAL2, GAL7, GAL10* or *MEL1* gene promoters.

According to this invention, the above strains are used in the synthesis of bioactive substances and other products of biotechnology.

Within the scope of this invention is the use of the above strains for the production of bioactive substances and other products of biotechnology. On the chromosome of these strains, the gene encoding the enzyme for the transformation of the transcription inductor into an inactive product is replaced by a gene encoding the transcription factor for the gatactose-inducible promoter, as described above.

The use of the above strains for the production of bioactive substances and other products of biotechnology, with the aid of said galactose-regulated strains, preferably of the family *Saccharomycetaceae* or *Cryptococcaceae*, preferably of the genus *Saccharomyces,* preferably *Saccharomyces cerevisiae.*

The use of the above strains for the production of bioactive substances and other products of biotechnology with the aid of said strains, characterized in that the gene encoding the enzyme for the transformation of the transcription inductor into an inactive product, is the *GAL1* gene replaced by the *GAL4* gene encoding the transcription factor.

The use of the above strains for the production of bioactive substances and other products of biotechnology with the aid of said strains, wherein the synthesis of the *GAL4* transcription factor is under the control of the galactose-inducible promoter, preferably under the control of the *GAL1* promoter.

The use of the above strains for the production of bioactive substances and other products of biotechnology with the aid of said strains, wherein the galactose-inducible promoter drives the expression of proteins in the presence of a transcription inductor, which is preferably chosen from *GAL1, GAL2, GAL7, GAL10* or *MEL1* gene promoters.

The use of the above strains for the production of bioactive substances and other products of biotechnology with the aid of said strains, which are hosts for plasmids, used in heterologous or homologous gene expression. Preferably, the plasmids are under the control of a galactose-inducible promoter, typically chosen from *GAL1, GAL2, GAL7, GAL10* or *MEL1* gene promoters.

The products of this invention obtainable with the use of highly-productive recombinant yeast strains, with modified galactose-regulated transcription; are preferably polypeptides, typically homologous and/or heterologous proteins.

The following description of materials and methods is only informative, and serves to illustrate the present invention. Other suitable materials and methods, obvious to those skilled in the art, may be utilized in manufacture.

The following examples represent and illustrate the present invention without, however, limiting the same in any way.

### Example 1: Construction of highly productive recombinant yeast Saccharomyces cerevisiae strain with chromosomal GAL1 by the GAL4 gene replacement

The present invention relates to the replacement of *GAL* genes on chromosome II in yeast *S*. *cerevisiae,* using cloning-free process, referred to as 'delitto perfetto' (Storici and co., 2001, Nature Biotechnology 19, 773-776). For the *GAL* gene replacement, other approach can also be used.

The *GAL1* inactivation and integration of *GAL4* were done as follows. Two long oligonucleotide primers were designed,
GAL1-core-S
(GTTAATATACCTCTATACTTTAACGTCAAGGAGAAAAAACTATAATGgagctcgttttcg acactgg) and
GAL1-core-AS
(GAAAAAAATGAGAAGTTGTTCTGAACAAAGTAAAAAAAAGAAGTATACtccttaccatt aagttgatc).
A counter-selectable reporter (CORE) cassette with K/URA3 (counter-selectable) and kanMX4 (reporter) was amplified as a 3.2 kb DNA fragment from pCORE (gift from F. Storici) using these 67- and 68-mers, comprising 20 bases homologous to the kanMX4 and K/URA markers, respectively, and 47 and 48 bases complementary to the regions upstream and downstream, respectively, of the yeast *GAL1* open reading frame (ORF). The CORE cassette was purified using QIAquick PCR Purification Kit and then transformed by standard recombinant techniques (Wach and co., 1994, Yeast 10, 1793-1804) into the commonly used BY4741 (MATa; his3Δ1; leu2Δ0; met15Δ0; ura3Δ0) yeast strain. Transformants were selected on YEPD (1% Bacto-pepton (Difco), 2% yeast extract, 2% glucose) plates containing 200 mg I⁻¹ of geneticin (G418). Clones with the correct CORE cassette integration were identified by colony PCR, using primers, designed for annealing sequences upstream and downstream from the integration locus and within the marker (CORE) cassette.
The following primers were used:
GAL1-A1 (GGCCCCACAAACCTTCAA),
GAL4-as (ATGTCAAGGTCTTCTCGAGG) and
K3 (AATATTGTTGATGCGCTGG).

*GAL4* gene was targeted into yeast strain with correct CORE cassette integration. To prevent non-specific annealing reactions, firstly a *GAL4*-long gene, containing 145 bp upstream and 533 bp downstream sequence of *GAL4* ORF was obtained. The *GAL4-*long gene (3.3 kbp) was amplified by PCR from yeast genomic DNA (from strain S288C: MAT-a; SUC2; mal; mel; gal2; CUP1; flo1; flo8-1). *GAL4* gene used for integration into the target locus was amplified from GAL4-long gene. Cells with correct CORE cassette integration were transformed with this PCR product and 5-fluoroorotic acid resistant (5FOA^{R}), G418 sensitive (G418^{S}) mutants were obtained. Clones with correct *GAL4* gene integration were identified by colony PCR, using primers for annealing a sequence upstream from the integration site, a sequence within the *kan*MX4 marker and a sequence within the *GAL4* gene. The following primer oligonucleotides were used:
GAL1-A1 (GGCCCCACAAACCTTCAA),
K3 (AATATTGTTGATGCGCTGG) and
GAL4-as (ATGTCAAGGTCTTCTCGAGG).

Sequence analysis provided additional confirmation that intact *GAL4* structural gene has been integrated accurately at the *GAL1* locus in the *GAL* recombinant strain. In first step, *GAL1* gene was successfully replaced by CORE cassette, which was, in second step, completely removed by *GAL4* open reading frame.

The present invention relates to the highly-productive recombinant strains from *Saccharomycetaceae* or *Cryptococcaceae* family, in which gene, encoding an enzyme that inactivates inductor galactose, has been replaced with a transcription activator of genes driven by galactose - inducible promoter. These isogenic strains are preferentially from *Saccharomyces* genus, preferentially from *Saccharomyces cerevisiae* species. Gene, encoding an enzyme relates to *GAL1,* gene encoding transcription activator relates to *GAL4* gene. Galactose-inducible promoter relates to *GAL1, GAL2, GAL7, GAL10* or *MEL1* gene promoter.

The present invention provides the recombinant strains that are host organisms for *GAL* promoter-driven expression of heterologous genes.

The present invention relates to the production of biological active compounds and other biotechnological products using presented strains that are preferentially from *Saccharomyces* genus, preferentially from *Saccharomyces cerevisiae* species. In described production, gene, encoding an enzyme relates to *GAL1,* gene encoding transcription activator relates to *GAL4* gene. Galactose-inducible promoter relates to *GAL1, GAL2, GAL7, GAL10 or MEL1* gene promoter.

To evaluate whether *GAL* gene replacement, relating to the invention, improve protein production, the level of green fluorescent protein (GFP) and Hepatitis B antigen (HbsAg) were analyzed in the *GAL* recombinant strain, relating to the invention, in *gal1* and in non-recombinant strains.

### Example 2: Expression of GFP

YCpGAL1-GFP and YEpGAL1-GFP were constructed as follows. To obtain YCpGAL1 and YEpGAL1, *GAL1* promoter and *ADH1* terminator were inserted into YCp111 and YEp181, respectively. Both shuttle vectors contain *LEU2* selection marker. YCp111 plasmid contains yeast autonomous replication (ARS1) and centromeric (CEN4) sequences. ARS/CEN vectors are mitotically highly stable, but the copy number is reduced to 1 or 2 per cell. GFP gene was excised from pGFP (Clontech Laboratories) with *Bam*HI, blunt-ended by Mung Bean Nuclease, digested with *Eco*RI and cloned into the appropriately treated YCpGAL1 vector. YEp181 is based on 2µ that enabling both mitotic stability and high vector copy number. YEpGAL1-GFP was constructed by cloning GFP into *Bam*HI/*Sma*I restricted YEpGAL1.

The resulting plasmids YCpGAL1-GFP and YEpGAL1-GFP, containing reporter gene, were transformed to the mutant strain, which relates to the invention, *gal1* and wild-type, respectively. Transformants were selected on minimal medium without leucine. The yeast cells were grown on YEPD medium, to mid-log phase and then transferred to medium containing 2 % galactose. The cells were induced for 18 hours at 30 °C, 180 rpm in a reciprocal shaker. For the estimation of GFP in viable cell, yeast were resuspended in TE buffer (10 mM Tris-HCl pH 8.0, 10 mM EDTA) to cell density 2×10⁷/mL. GFP fluorescence intensity was measured using a fluorescence spectrophotometer at an excitation wavelength of 395 nm and an emission wavelength of 509 nm, respectively (5-nm slit widths). A standard dilution series of GFP provided by Clontech was made in TE buffer and standard curves with a high degree of confidence were established. Standards and samples were measured in triplicate and averaged. All samples were appropriately diluted to give a reading within the range of the standard curve. The level of protein synthesis was determined in the recombinant strain, relating to the invention, in *gal1* and in non-recombinant strain.

### 1. Expression of GFP in yeast bearing YCpGAL1-GFP

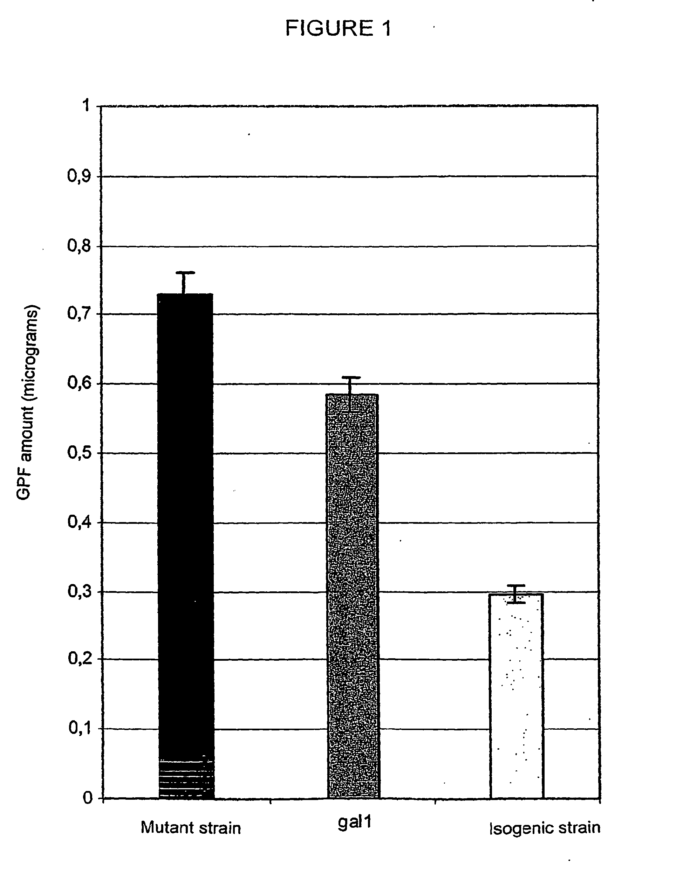

The level of recombinant protein was determined in recombinant strain relating to the invention, in *gal1* and in non-recombinant strains. The yeast were cultured and induced as described above. The level of GFP was quantified from established standard curves. Experiments were performed in triplicates. In yeast containing the low-copy plasmid there was an approximately 96 % increase in GFP production in the *gal1* mutant strain. Moreover, in the resulting recombinant cells that relate to the invention a 145 % increase in fluorescence relative to the wild-type was observed. The GFP production in 2×10⁷ cells were 0.297 µg in wild-type, 0.585 µg in *gal1* mutant, and 0.729 µg in mutant' strain (Figure 1).

### 2. Expression of GFP in yeast bearing YEpGAL1-GFP

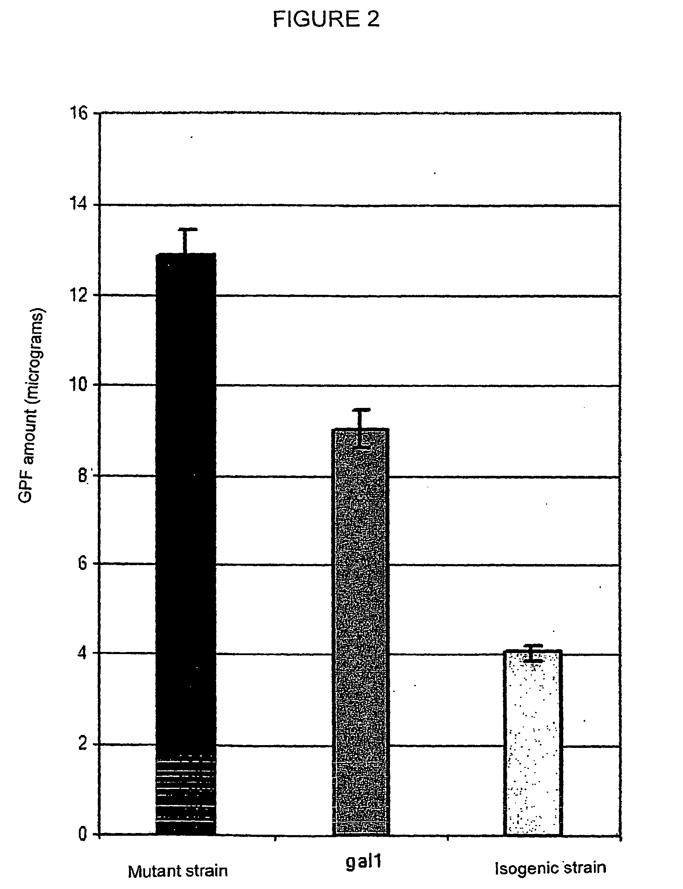

The level of GFP in yeast strains bearing multi-copy plasmid was quantified. The yeast were cultured and induced as described above. Data are the mean from three separate experiments. In yeast containing the multi-copy plasmid there was an approximately 123 % increase in GFP production in the *gal1* mutant strain. Moreover, in the resulting recombinant cells that relate to the invention a 218 % increase in fluorescence relative to the wild-type was observed. The GFP production in 2×10⁷ cells were 4.05 µg in wild-type, 9.03 µg in *gal1* mutant, and 12.89 µg in mutant strain (Figure 2).

### Example 3: Expression of HBsAg

Multi-copy plasmid, bearing gene for Hepatitis B Antigen (HbsAg), were constructed as follows. *GAL1* promoter and *ADH1* terminator were inserted into YEp181 shuttle vector. Vector was digested with *Bam*HI, blunt-ended by Klenow fragment and subsequently digested with *Kpn*I*.* HBsAg gene was amplified from pRC/CMV-HBs(S) (gift from Aldevron) by PCR and cloned into expression vector. The resulting plasmid, containing gene for HBsAg, were transformed to the mutant strain, which relates to the invention, *gal1* and wild-type, respectively. Transformants were selected on minimal medium without leucine. The yeast cells were grown on YEPD medium, to mid-log phase and then transferred to medium containing 2 % galactose. The cells were induced for 18 hours at 30 °C, 180 rpm in a reciprocal shaker. Aliquots of cells corresponding to 50 absorbance unit (OD) were collected, washed twice with ice-cold lysis buffer (10 mM PBS pH 7.2, 5 mM EDTA, 0.5 M NaCl, 0.1 % (v/v) Triton X-100, 1 mM PMSF) and lysed with glass beads (0,45 µm). Protein concentration was determined by Bradford method with bovine serum albumin as a standard. To estimate the total amount of HbsAg, quantitative dot-blot assay were performed. We used mouse antibody raised against Hepatitis B Surface Antigen (provided by Aldevron). Comparing to commercial available HbsAg standard, total quantity of recombinant protein in yeast transformants was determined. Upon induction by galactose, there was an approximately 2-fold increase in HbsAg production in *gal1* mutant strain. Moreover, in the resulting recombinant strain that relates to the invention a 3-fold increase in protein production was observed.

## Claims

1. Highly productive recombinant yeast strains with modified galactose-regulated transcription, **characterized in that** on the chromosome the *GAL* 1 gene encoding the enzyme for the transformation of the transcription inductor into an inactive product is replaced by the *GAL4* gene encoding the transcription factor for the galactose-inducible promoter.

2. Strains as claimed in claim 1, **characterized in that** the isogenic strains are chosen from the family *Saccharomycetaceae* or *Cryptococcaceae.*

3. Strains as claimed in claim 1 or 2, **characterized in that** the isogenic strains are of the genus *Saccharomyces*.

4. Strains as claimed in any one of claims 1 to 3, **characterized in that** the isogenic strains are of the species *Saccharomyces cerevisiae*

5. Strains as claimed in any one of claims 1 to 4, **characterized in that** the synthesis of the *GAL4* transcription factor is under the control of the galactose-inducible promoter.

6. Strains as claimed in any one of claims 1 to 5, **characterized in that** the synthesis of the *GAL4* transcription factor is under the control of *GAL1, GAL2, GAL7, GAL10* or *MEL1* promoters.

7. Strains as claimed in any one of claims 1 to 6, **characterized in that** the synthesis of the *GAL4* transcription factor is under the control of the *GAL* 1 promoter.

8. Strains as claimed in any one of claims 1 to 4, **characterized in that** the galactose-inducible promoter drives the expression of proteins in the presence of a transcription inductor.

9. Strains as claimed in claim 8, **characterized in that** the galactose-inducible promoter is chosen from *GAL1*, *GAL2, GAL7, GAL10* or *MEL1* gene promoters.

10. Strains as claimed in any one of claims 1 to 9, **characterized in that** they are hosts for plasmids, used in heterologous or homologous gene expression and/or in the synthesis of bioactive substances and other products of biotechnology.

11. Strains as claimed in claim 10, **characterized in that** they are hosts for plasmids, used in heterologous or homologous gene expressions under the control of the galactose-inducible promoter.

12. Strains as claimed in claims 10 or 11, **characterized in that** they are hosts for plasmids, used in heterologous or homologous gene expressions under the control of the galactose-inducible promoter, chosen from *GAL1, GAL2, GAL7, GAL10* or *MEL1* gene promoters.

13. Strains as claimed in any of claims 1 to 12, **characterized in that** they are used in the synthesis of bioactive substances and other products of biotechnology.

14. A use of highly productive recombinant yeast strains with modified galactose-regulated transcription for the production of bioactive substances and other products of biotechnology, **characterized in that** on the chromosome the *GAL1* gene encoding the enzyme for the transformation of the transcription inductor into an inactive product is replaced by the *GAL4* gene encoding the transcription factor for the galactose-inducible promoter, as described in any of claims 1 to 13.

15. The use as claimed in claim 14, **characterized in that** the utilized strains are galactose-regulated strains.

16. The use as claimed in claim 14 or 15, **characterized in that** the strains are chosen from the family *Saccharomycetaceae* or *Cyptococcaceae.*

17. The use as claimed in any one of claims 14 to 16, **characterized in that** the strains are of the genus *Saccharomyces.*

18. The use as claimed in any one of claims 14 to 17, **characterized in that** the strains are of the species *Saccharomyces cerevisiae.*

19. The use as claimed in any one of claims 14 to 18, **characterized in that** the synthesis of the GAL4 transcription factor is under the control of the galactose-inducible promoter.

20. The use as claimed in any one of claims 14 to 19, **characterized in that** the synthesis of the *GAL4* transcription factor is under the control of the *GAL1* promoter.

21. The use as claimed in any one of claims 14 to 18, **characterized in that** the galactose-inducible promoter drives the expression of proteins in the presence of a transcription inductor.

22. The use as claimed in claim 21, **characterized in that** the galactose-inducible promoter is chosen from *GAL1, GAL2, GAL7, GAL10* or *MEL1* gene promoters.

23. The use as claimed in any one of claims 14 to 21, **characterized in that** the strains as claimed in claims 1 to 10 are hosts for plasmids, used in heterologous or homologous gene expression.

24. The use as claimed in claim 23, **characterized in that** the strains as claimed in claims 1 to 10 are hosts for plasmids, used in heterologous or homologous gene expressions under the control of the galactose-inducible promoter.

25. The use as claimed in claim 23 or 24, **characterized in that** the strains as claimed in claims 1 to 10 are hosts for plasmids, used in heterologous or homologous gene expressions under the control of the galactose-inducible promoter, chosen from *GAL 1, GAL2, GAL7, GAL 10* or *MEL1* gene promoters.

26. A method of producing a bioactive substance or another product of biotechnology, wherein the bioactive substance or the product of biotechnology is produced by a highly productive recombinant yeast strain with modified galactose-regulated transcription of any one of claims 1 to 13.

## Patentansprüche

1. Hoch produktive rekombinante Hefestämme mit modifizierter Galaktose-regulierter Transkription, **dadurch gekennzeichnet, dass** im Chromosom das *GAL1*-Gen, welches das Enzym für die Transformation des Transkriptionsinduktors in ein inaktives Produkt kodiert, durch das *GAL4*-Gen, welches den Transkriptionsfaktor für den Galaktose-induzierbaren Promotor kodiert, ersetzt ist.

2. Stämme wie beansprucht nach Anspruch 1, **dadurch gekennzeichnet, dass** die isogenen Stämme ausgewählt sind aus der Familie *Saccharomycetaceae* oder *Cryptococcaceae.*

3. Stämme wie beansprucht nach Anspruch 1 oder 2, **dadurch gekennzeichnet, dass** die isogenen Stämme vom Genus *Saccharomyces* sind.

4. Stämme wie beansprucht nach einem der Ansprüche 1 bis 3, **dadurch gekennzeichnet, dass** die isogenen Stämme von der Spezies *Saccharomyces cerevisiae* sind.

5. Stämme wie beansprucht nach einem der Ansprüche 1 bis 4, **dadurch gekennzeichnet, dass** die Synthese des *GAL4*-Transkriptionsfaktors unter der Kontrolle des Galaktose-induzierbaren Promotors steht.

6. Stämme wie beansprucht nach einem der Ansprüche 1 bis 5, **dadurch gekennzeichnet, dass** die Synthese des *GAL4*-Transkriptionsfaktors unter der Kontrolle des *GAL1-, GAL2-, GAL7-, GAL10-* oder *MEL1*-Promotors steht.

7. Stämme wie beansprucht nach einem der Ansprüche 1 bis 6, **dadurch gekennzeichnet, dass** die Synthese des *GAL4*-Transkriptionsfaktors unter der Kontrolle des *GAL1-*Promotors steht.

8. Stämme wie beansprucht nach einem der Ansprüche 1 bis 4, **dadurch gekennzeichnet, dass** der Galaktose-induzierbare Promotor die Expression von Proteinen in Gegenwart eines Transkriptionsinduktors antreibt.

9. Stämme wie beansprucht nach Anspruch 8, **dadurch gekennzeichnet, dass** der Galaktose-induzierbare Promotor ausgewählt ist aus *GAL1-, GAL2-, GAL7-, GAL10-* oder *MEL1-*Gen-Promotoren.

10. Stämme wie beansprucht nach einem der Ansprüche 1 bis 9, **dadurch gekennzeichnet, dass** sie Wirte für Plasmide sind, welche verwendet werden bei heterologer oder homologer Genexpression und/oder bei der Synthese von bioaktiven Substanzen und anderen biotechnologischen Produkten.

11. Stämme wie beansprucht nach Anspruch 10, **dadurch gekennzeichnet, dass** sie Wirte für Plasmide sind, welche verwendet werden bei heterologen oder homologen Genexpressionen unter der Kontrolle des Galaktose-induzierbaren Promotors.

12. Stämme wie beansprucht nach Anspruch 10 oder 11, **dadurch gekennzeichnet, dass** sie Wirte für Plasmide sind, welche verwendet werden bei heterologen oder homologen Genexpressionen unter der Kontrolle des Galaktose-induzierbaren Promotors, welcher ausgewählt ist aus *GAL1*-, *GAL2-, GAL7-, GAL10*- oder *MEL1*-Gen-Promotoren.

13. Stämme wie beansprucht nach einem der Ansprüche 1 bis 12, **dadurch gekennzeichnet, dass** sie bei der Synthese von bioaktiven Substanzen und anderen biotechnologischen Produkten verwendet werden.

14. Verwendung von hochproduktiven rekombinanten Hefestämmen mit modifizierter Galaktose-regulierter Transkription für die Herstellung von bioaktiven Substanzen und anderen biotechnologischen Produkten, **dadurch gekennzeichnet, dass** im Chromosom das *GAL1*-Gen, welches das Enzym für die Transformation des Transkriptionsinduktors in ein inaktives Produkt kodiert, durch das *GAL4*-Gen, welches den Transkriptionsfaktor für den Galaktose-induzierbaren Promotor kodiert, ersetzt ist, wie beschrieben in einem der Ansprüche 1 bis 13.

15. Verwendung wie beansprucht nach Anspruch 14, **dadurch gekennzeichnet, dass** die verwendeten Stämme Galaktose-regulierte Stämme sind.

16. Verwendung wie beansprucht nach Anspruch 14 oder 15, **dadurch gekennzeichnet, dass** die Stämme ausgewählt sind aus der Familie *Saccharomycetaceae* oder *Cryptococcaceae.*

17. Verwendung wie beansprucht nach einem der Ansprüche 14 bis 16, **dadurch gekennzeichnet, dass** die Stämme vom Genus *Saccharomyces* sind.

18. Verwendung wie beansprucht nach einem der Ansprüche 14 bis 17, **dadurch gekennzeichnet, dass** die Stämme von der Spezies *Saccharomyces cerevisiae* sind.

19. Verwendung wie beansprucht nach einem der Ansprüche 14 bis 18, **dadurch gekennzeichnet, dass** die Synthese des *GAL4*-Transkriptionsfaktors unter der Kontrolle des Galaktose-induzierbaren Promotors steht.

20. Verwendung wie beansprucht nach einem der Ansprüche 14 bis 19, **dadurch gekennzeichnet, dass** die Synthese des *GAL4*-Transkriptionsfaktors unter der Kontrolle des *GAL1*-Promotors steht.

21. Verwendung wie beansprucht nach einem der Ansprüche 14 bis 18, **dadurch gekennzeichnet, dass** der Galaktose-induzierbare Promotor die Expression von Proteinen in Gegenwart eines Transkriptionsinduktors antreibt.

22. Verwendung wie beansprucht nach Anspruch 21, **dadurch gekennzeichnet, dass** der Galaktose-induzierbare Promotor ausgewählt ist aus *GAL1-, GAL2-, GAL7-, GAL10-* oder *MEL1*-Gen-Promotoren.

23. Verwendung wie beansprucht nach einem der Ansprüche 14 bis 21, **dadurch gekennzeichnet, dass** die Stämme wie beansprucht nach den Ansprüchen 1 bis 10 Wirte sind für Plasmide, welche bei heterologen oder homologen Genexpressionen verwendet werden.

24. Verwendung wie beansprucht nach Anspruch 23, **dadurch gekennzeichnet, dass** die Stämme wie beansprucht nach den Ansprüchen 1 bis 10 Wirte sind für'Plasmide, welche verwendet werden bei heterologen oder homologen Genexpressionen unter der Kontrolle des Galaktose-induzierbaren Promotors.

25. Verwendung wie beansprucht nach Anspruch 23 oder 24, **dadurch gekennzeichnet, dass** die Stämme wie beansprucht nach den Ansprüchen 1 bis 10 Wirte sind für Plasmide, welche verwendet werden bei heterologen oder homologen Genexpressionen unter der Kontrolle des Galaktose-induzierbaren Promotors, welcher ausgewählt ist aus *GAL1-, GAL2-, GAL7-, GAL10-* oder *MEL1*-Gen-Promotoren.

26. Verfahren zur Herstellung einer bioaktiven Substanz oder eines anderen biotechnologischen Produktes, wobei die bioaktive Substanz oder das biotechnologische Produkt hergestellt wird mittels eines hoch produktiven rekombinanten Hefestammes mit modifizierter Galaktose-regulierter Transkription nach einem der Ansprüche 1 bis 13.

## Revendications

1. Souches de levure recombinées à haut rendement avec transcription régulée par galactose modifiée, **caractérisées en ce que** sur le chromosome, le gène *GAL1* codant l'enzyme pour la transformation de l'inducteur de transcription en un produit inactif est remplacé par le gène *GAL4* codant le facteur de transcription pour le promoteur inductible par le galactose.

2. Souches telles que revendiquées dans la revendication 1, **caractérisées en ce que** les souches isogéniques sont choisies dans la famille *Saccharomycetaceae* ou *Cryptococcaceae.*

3. Souches telles que revendiquées dans la revendication 1 ou 2, **caractérisées en ce que** les souches isogéniques sont du genre *Saccharomyces.*

4. Souches telles que revendiquées dans l'une quelconque des revendications 1 à 3, **caractérisées en ce que** les souches isogéniques sont de l'espèce *Saccharomyces cerevisiae.*

5. Souches telles que revendiquées dans l'une quelconque des revendications 1 à 4, **caractérisées en ce que** la synthèse du facteur de transcription *GAL4* est sous le contrôle du promoteur inductible par le galactose.

6. Souches telles que revendiquées dans l'une quelconque des revendications 1 à 5, **caractérisées en ce que** la synthèse du facteur de transcription *GAL4* est sous le contrôle des promoteurs *GAL1, GAL2, GAL7, GAL10* ou *MEL1.*

7. Souches telles que revendiquées dans l'une quelconque des revendications 1 à 6, **caractérisées en ce que** la synthèse du facteur de transcription *GAL4* est sous le contrôle du promoteur *GAL1.*

8. Souches telles que revendiquées dans l'une quelconque des revendications 1 à 4, **caractérisées en ce que** le promoteur inductible par le galactose stimule l'expression des protéines en présence d'un inducteur de transcription.

9. Souches telles que revendiquées dans la revendication 8, **caractérisées en ce que** le promoteur inductible par le galactose est choisi dans les promoteurs de gènes *GAL1, GAL2, GAL7, GAL10* ou *MEL1.*

10. Souches telles que revendiquées dans l'une quelconque des revendications 1 à 9, **caractérisées en ce qu'**elle servent d'hôtes pour les plasmides, utilisés dans l'expression des gènes hétérologues ou homologues et/ou dans la synthèse de substances bioactives et d'autres produits de biotechnologie.

11. Souches telles que revendiquées dans la revendication 10, **caractérisées en ce qu'**elles servent d'hôtes aux plasmides, utilisés dans les expressions de gènes hétérologues ou homologues sous le contrôle du promoteur inductible par le galactose.

12. Souches telles que revendiquées dans les revendications 10 ou 11, **caractérisées en ce qu'**elles servent d'hôtes pour les plasmides, utilisés dans les expressions de gènes hétérologues ou homologues sous le contrôle du promoteur inductible par le galactose, choisi dans les promoteurs de gènes *GAL1, GAL2, GAL7, GAL10* ou *MEL1.*

13. Souches telles que revendiquées dans l'une quelconque des revendications 1 à 12, **caractérisées en ce qu'**elles sont utilisées dans la synthèse de substances bioactives et d'autres produits de biotechnologie.

14. Utilisation de souches de levure recombinées à haut rendement avec transcription régulée par galactose modifiée pour la production de substances bioactives et d'autres produits de biotechnologie, **caractérisée en ce que** sur le chromosome, le gène *GAL1* codant l'enzyme pour la transformation de l'inducteur de transcription en un produit inactif est remplacé par le gène *GAL4* codant le facteur de transcription pour le promoteur inductible par le galactose, tel que décrit dans l'une quelconque des revendications 1 à 13.

15. Utilisation telle que revendiquée dans la revendication 14, **caractérisée en ce que** les souches utilisées sont des souches régulées par le galactose.

16. Utilisation telle que revendiquée dans la revendication 14 ou 15, **caractérisée en ce que** les souches sont choisies dans la famille *Saccharomycetaceae* ou *Cryptococcaceae.*

17. Utilisation telle que revendiquée dans l'une quelconque des revendications 14 à 16, **caractérisée en ce que** les souches sont du genre *Saccharomyces.*

18. Utilisation telle que revendiquée dans l'une quelconque des revendications 14 à 17, **caractérisée en ce que** les souches sont de l'espèce *Saccharomyces cerevisiae.*

19. Utilisation telle que revendiquée dans l'une quelconque des revendications 14 à 18, **caractérisée en ce que** la synthèse du facteur de transcription *GAL4* est sous le contrôle du promoteur inductible par le galactose.

20. Utilisation telle que revendiquée dans l'une quelconque des revendications 14 à 19, **caractérisée en ce que** la synthèse du facteur de transcription *GAL4* est sous le contrôle du promoteur *GAL1.*

21. Utilisation telle que revendiquée dans l'une quelconque des revendications 14 à 18, **caractérisée en ce que** le promoteur inductible par le galactose stimule l'expression des protéines en présence d'un inducteur de transcription.

22. Utilisation telle que revendiquée dans la revendication 21, **caractérisée en ce que** le promoteur inductible par le galactose est choisi dans les promoteurs de gènes *GAL1, GAL2, GAL7, GAL10* ou *MEL1.*

23. Utilisation telle que revendiquée dans l'une quelconque des revendications 14 à 21, **caractérisée en ce que** les souches telles que revendiquées dans les revendications 1 à 10 servent d'hôtes pour les plasmides, utilisés dans l'expression des gènes hétérologues ou homologues.

24. Utilisation telle que revendiquée dans la revendication 23, **caractérisée en ce que** les souches telles que revendiquées dans les revendications 1 à 10 servent d'hôtes pour les plasmides, utilisés dans les expressions des gènes hétérologues ou homologues sous le contrôle du promoteur inductible par le galactose.

25. Utilisation telle que revendiquée dans la revendication 23 ou 24, **caractérisée en ce que** les souches telles que revendiquées dans les revendications 1 à 10 servent d'hôtes pour les plasmides, utilisés dans les expressions des gènes hétérologues ou homologues sous le contrôle du promoteur inductible par le galactose, choisi dans les promoteurs de gènes *GAL1, GAL2, GAL7, GAL10* ou *MEL1.*

26. Procédé de production d'une substance bioactive ou d'un autre produit de biotechnologie, dans lequel la substance bioactive ou le produit de biotechnologie est produit par une souche de levure recombinée à haut rendement avec transcription régulée par le galactose modifiée selon l'une quelconque des revendications 1 à 13.
